# EUROPEAN PATENT APPLICATION

(11) **EP 3 978 067 A2**
(43) Date of publication of application: **06.04.2022**
(21) Application number: 20814406.3
(22) Date of filing: 20.05.2020
(51) Int. Cl.: A61N 1/05

(54) **HIS BUNDLE-DETECTING SNARE CATHETER**

(30) Priority: 31.05.2019 KR 20190064235
(71) Applicant: Tau-PNU Medical Co., Ltd., Busan 46241 (KR)
(72) Inventor: KIM, June Hong, Busan 48516 (KR); PARK, Kyone Peter, Yangsan-si Gyeongsangnam-do 50653 (KR)
(74) Representative: dompatent von Kreisler Selting Werner - Partnerschaft von Patent- und Rechtsanwälten mbB
(86) International application number: PCT/KR2020/006604
(87) International publication number: WO 2020/242120

(57) **Abstract**

A His bundle-detecting snare catheter according to the present invention detects a myocardial electrical signal and locates the position of a His bundle through the insertion of a snare and an EP catheter into a dual lumen catheter having two lumens, and captures a cerclage wire, which has passed through the His bundle, and safely guides same into a body. The His bundle-detecting snare catheter according to the present invention comprises: a dual lumen catheter having formed therein a first lumen and a second lumen; an electrical signal detection catheter which is inserted into the first lumen or the second lumen and detects a myocardial electrical signal; and a snare which is inserted into the first lumen or the second lumen and has one or more annular wires at a distal portion thereof, wherein the electrical signal detection catheter and the snare are inserted into different lumens.

## Description

### [Technical Field]

The present invention relates to a HIS-bundle-detecting snare catheter, and more particularly, to a HIS-bundle-detecting snare catheter in which a snare and an electrophysiology (EP) catheter are inserted into a dual lumen catheter including two lumens formed therein, an electrical signal of myocardia is detected to locate a His bundle, and a cerclage wire having passed through the His bundle is captured and safely induced into a human body.

### [Background Art]

A cardiac conduction system is formed from a sinoatial node in an atrium via an atrioventricular node and is divided into a right bundle and a left bundle in a His bundle of a ventricle through Purkinje fibers.

In an electrocardiogram, a QRS wave is generated due to a depolarization process of ventricular muscles. Here, an initial downward wave following a P-wave is referred to as a Q wave, an initial upward wave is referred to as an R wave, and a downward wave following the R wave is referred to as an S wave. A width of QRS means time in which electricity is conducted from a His bundle to all ventricles. In a normal case, the width of QRS wave is about 0.12 seconds (about 90 ms). When more than 0.12 seconds (120 ms), intraventricular conduction disturbance is implied. When an electrical conduction time is long, the width of QRS increases. When the electrical conduction time is short, the width of QRS decreases. Wide QRS causes ventricular desynchronization in which ventricular movements are not synchronized such that a ventricular failure is caused.

When malfunction of an electricity delivery system itself of the heart occurs or abnormal electricity occurs out of the system and is delivered to another part, arrhythmia occurs.

Methods of diagnosing patients with cardiac arrhythmia include electrocardiography, Holter monitoring, an electrophysiological test, and the like.

Among them, the electrophysiological test measures and records electrical activity at a variety of parts of the heart while a catheter on which an electrode is mounted is inserted into the heart through blood vessels of an arm or leg or to monitor cardiac responses while electrical stimuli are given to a variety of parts of the heart. Through the electrophysiological test, a cardiac surgeon may locate a precise cause of arrhythmia occurrence, performs treatment of removing the located cause, or treats arrhythmia by installing a pacemaker.

As a part of such research, the present inventor has filed applications for a method, device, and catheter for locating an end of a lead of a pacemaker which has passed through sinus coronaries in a ventricular septum which are disclosed in Korean Patent Publication Nos. 10-2016-0011530 and 10-2016-0020887. The published applications have been filed by improving a cerclage wire disclosed in Korean Patent Registration No. 10-1116867 filed by the present inventor and relate to an apparatus configured to more simply fix a pacemaker to the heart in comparison to conventional techniques.

The published application discloses a method including introducing a surgical wire through a superior vena cava and a coronary sinus, passing the surgical wire through an intermediate or cacuminal inter-ventricular septum, and then guiding the surgical wire toward the superior vena cava or inferior vena cava and placing an end of a lead of a pacemaker inside the inter-ventricular septum by inserting the lead of the pacemaker along the surgical wire.

Here, when the end of the lead of the pacemaker is fixed to a part close to a His bundle located in the inter-ventricular septum and electrical stimuli are applied, an electrical conduction time may be reduced and narrow QRS may be obtained.

Accordingly, research on an apparatus configured to induce a lead of a pacemaker to a His bundle so as to allow the lead of the pacemaker to apply electrical stimuli to a part close to the His bundle is necessary.

### [Disclosure]

### [Technical Problem]

The present invention is directed to providing a HIS-bundle-detecting snare catheter configured to install a pacemaker at a place closer to a His bundle in order to increase an arrhythmia treatment effect in which the HIS-bundle-detecting snare catheter configured to insert an electrophysiology (EP) catheter configured to see a position of the His bundle by detecting an electrical signal of myocardia and a snare configured to capture a cerclage wire are inserted simultaneously into a human body so as to capture and move the cerclage wire having passed through the His bundle to the outside of the human body.

The present invention is also directed to providing a HIS-bundle-detecting snare catheter configured to easily sense a His bundle and to move a catheter to a desired position so as to capture a cerclage wire having passed through the His bundle.

Objects of the present invention are not limited to the above-described objects and other unstated objects of the present invention will be understood clearly by one of ordinary skill in the art from the following descriptions.

### [Technical Solution]

One aspect of the present invention provides a HIS-bundle-detecting snare catheter including a dual lumen catheter including a first lumen and a second lumen which are formed therein, an electrical-signal-detecting catheter inserted into the first lumen or the second lumen to detect an electrical signal of myocardia, and a snare inserted into the first lumen or the second lumen and including one or more annular wire at a distal part. Here, the electrical-signal-detecting catheter and the snare are inserted into different lumens, respectively.

Here, the dual lumen catheter may include a bending portion at the distal part to be bent at a predetermined angle, and the dual lumen catheter may include a position adjustor installed on a proximal part so as to adjust a position of the distal part of the dual lumen catheter when the position adjustor is adjusted.

The dual lumen catheter may include a hole formed in a side of the distal part. Also, the hole may be coupled to and communicate with an end of a distal part of the first lumen or the second lumen, and the electrical-signal-detecting catheter or the snare may protrude through the hole.

The snare may include a capture portion including one or more annular wires and a body portion having one end coupled to the annular wire and another end passing through the snare lumen and located outside a human body. Also, the other end of the body portion may be pushed or pulled to allow the capture portion of the snare to be inserted into or protrude from the dual lumen catheter.

A guide wire may be inserted into the first lumen or the second lumen, and the guide wire may be inserted into the human body in advance of the HIS-bundle-detecting snare catheter to allow the HIS-bundle-detecting snare catheter to easily move to the human body.

The HIS-bundle-detecting snare catheter may include a hemostasis valve on an end of a proximal end of the position adjustor to prevent blood flowing into the dual lumen catheter from leaking when the HIS-bundle-detecting snare catheter is inserted into the human body.

### [Advantageous Effects]

According to the present invention, in a HIS-bundle-detecting snare catheter, an electrical-signal-detecting catheter and a snare are inserted into a dual lumen catheter simultaneously to detect an electrical signal of myocardia so as to allow a cerclage wire to pass through a position of myocardia desired by a user and to easily capture and move the cerclage wire having passed through a His bundle to the inferior vena cava.

Also, the HIS-bundle-detecting snare catheter according to the present invention may move a distal part of the dual lumen catheter to a desired position using a position adjustor so that the electrical-signal-detecting catheter and the snare may protrude to be closer to the myocardia.

### [Description of Drawings]

FIG. 1 is a perspective view of a HIS-bundle-detecting snare catheter according to a first exemplary embodiment of the present invention.
FIG. 2 is a perspective view of a HIS-bundle-detecting snare catheter according to a second exemplary embodiment of the present invention.
FIG. 3 is a perspective view of a HIS-bundle-detecting snare catheter according to a third exemplary embodiment of the present invention.
FIG. 4 is a perspective view of a HIS-bundle-detecting snare catheter according to a fourth exemplary embodiment of the present invention.
FIG. 5 is a flowchart illustrating a method of performing treatment using the HIS-bundle-detecting snare catheter according to the present invention.

### <Description of Reference Numerals>

10: dual lumen catheter
12: first lumen
14: second lumen
20: snare
22: capture portion
24: body portion
30: electrical-signal-detecting catheter
32: electrode
40: position adjusting device
42: hemostasis valve
50: cerclage wire

### [Modes of the Invention]

Hereinafter, exemplary embodiments of the present invention will be described in detail with reference to the attached drawings.

A HIS-bundle-detecting snare catheter according to the present invention is a device configured so that a cerclage wire (treatment wire) inserted into a human body before a lead of a pacemaker passes through a His bundle and is easily induced into inferior vena cava to allow an end of the lead of the pacemaker to be fixed to the His bundle so as to reduce an electrical conducting time and to increase an arrhythmia treatment effect.

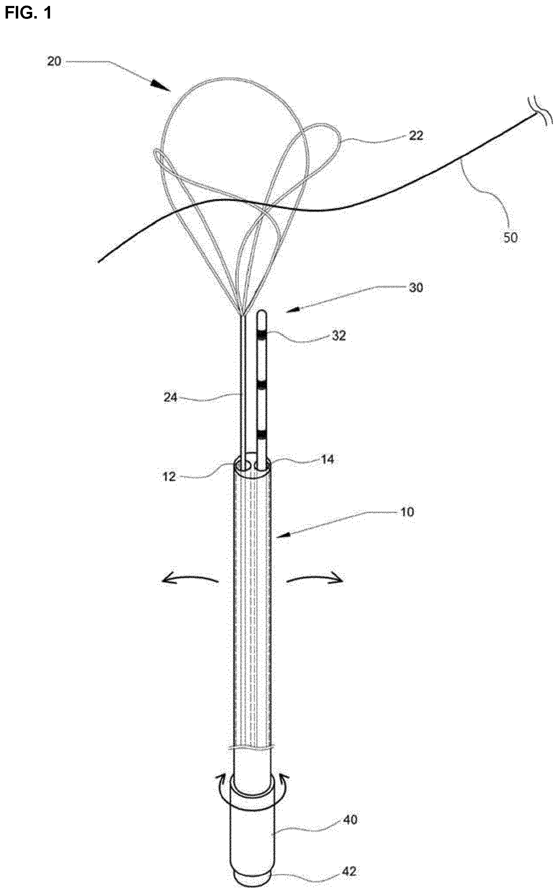

FIG. 1 is a perspective view of a HIS-bundle-detecting snare catheter according to a first exemplary embodiment of the present invention. Referring to FIG. 1, the HIS-bundle-detecting snare catheter according to the first exemplary embodiment of the present invention includes a dual lumen catheter 10, a snare 20, and an electrical-signal-detecting catheter 30.

The dual lumen catheter 10 has a cylindrical shape that is an exterior of a general catheter and includes two lumens including a first lumen 12 and a second lumen 14 which are two lumens into which a tool is insertable and are called a dual lumen catheter.

The dual lumen catheter 10 may be formed of a synthetic resin material such as a ductile rubber material, a ductile plastic material, and the like which are soft and have high elasticity and restorability.

The first lumen 12 and the second lumen 14 may be formed to be parallel at a certain interval, have diameters of 6 Fr (French) or less, and have the same diameters or different diameters.

The first lumen 12 and the second lumen 14 have distal ends formed on a distal end of the dual lumen catheter 10 so that the snare 20 or the electrical-signal-detecting catheter 30 are inserted into the dual lumen catheter 10 and protrude from the distal end of the dual lumen catheter 10.

The snare 20 includes a capture portion 22 including one or more annular wires and a body portion 24 having one end coupled to the capture portion 22 and the other end inserted into the first lumen 12 or the second lumen 14. The snare 20 may employ metal materials (SUS304, nitinol, titanium, and the like) or the like which are insertable into a human body.

The capture portion 22 includes one or more annular wires having both ends coupled to one end of the body portion 24 and may include three or four annular wires. The capture portion 22 is formed of a shape memory alloy, an elastic body, or a selfexpandable stent and has a feature of retracting while being inserted into the first lumen 12 or the second lumen 14 and extends while protruding therefrom.

In more detail, the snare 20 pushes the body portion 24 while moving according to pushing or pulling one side of the body portion 24 so as to be inserted into the first lumen 12 or the second lumen 14 and protrudes and extends to the outside of the dual lumen catheter 10. When a cerclage wire 50 having passed through the His bundle is located on the snare 20, the cerclage wire 50 is captured by pulling the body portion 24 and inserted into the dual lumen catheter 10.

The electrical-signal-detecting catheter 30 includes a plurality of electrodes 32 and may be inserted into the first lumen 12 or the second lumen 14. Here, the snare 20 and the electrical-signal-detecting catheter 30 may be inserted into different lumens.

Accordingly, the snare 20 and the electrical-signal-detecting catheter 30 may be inserted into the dual lumen catheter 10 simultaneously. A position of the His bundle may be detected using the electrical-signal-detecting catheter 30 and the cerclage wire having passed through the His bundle may be captured and withdrawn to inferior vena cava using the snare 20.

The electrodes 32 are configured to detect an electrical signal of myocardia. One or more electrodes 32 may be coupled to an outer circumferential surface of the electrical-signal-detecting catheter 30. In more detail, the plurality of electrodes 32 formed to have a strand shape are installed to be spaced at certain intervals on the outer circumferential surface of the electrical-signal-detecting catheter 30. Using the plurality of electrodes 32, electrical signals at a variety of positions may be detected and the His bundle may be located.

The dual lumen catheter 10 includes a position adjustor 40 installed on a proximal part. The position adjustor 40 may rotate in one direction. A distal part of the dual lumen catheter 10 may be moved leftward or rightward and moved to a myocardiac position desired by a practitioner by adjusting the position adjustor 40 so that the snare 20 and the electrical-signal-detecting catheter 30 protrude toward a place near myocardia.

The position adjustor 40 is formed by coupling a hemostasis valve 42 to a proximal end to block the proximal end of the position adjustor 40 and prevents blood flowing from the dual lumen catheter 10 into the position adjustor 40 from leaking through the proximal end of the position adjustor 40. The hemostasis valve 42 may be formed of a silicone material but is not limited thereto.

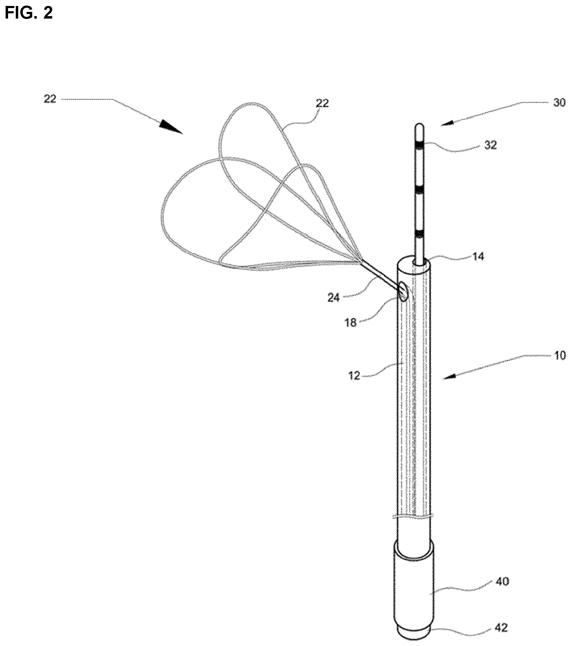

FIG. 2 is a perspective view of a HIS-bundle-detecting snare catheter according to a second exemplary embodiment of the present invention.

In the HIS-bundle-detecting snare catheter according to the second exemplary embodiment of the present invention, the dual lumen catheter 10 includes a hole 18 formed in a side of a distal part, and the hole 18 is coupled to and communicates with a distal end of the first lumen 12.

In other words, the distal end of the first lumen 12 is located on a side of the distal part of the dual lumen catheter 10, and a distal end of the second lumen 14 is located on the distal end of the dual lumen catheter 10.

Accordingly, the snare 20 or the electrical-signal-detecting catheter 30 which is inserted into the first lumen 12 protrudes toward the side of the distal part of the dual lumen catheter 10.

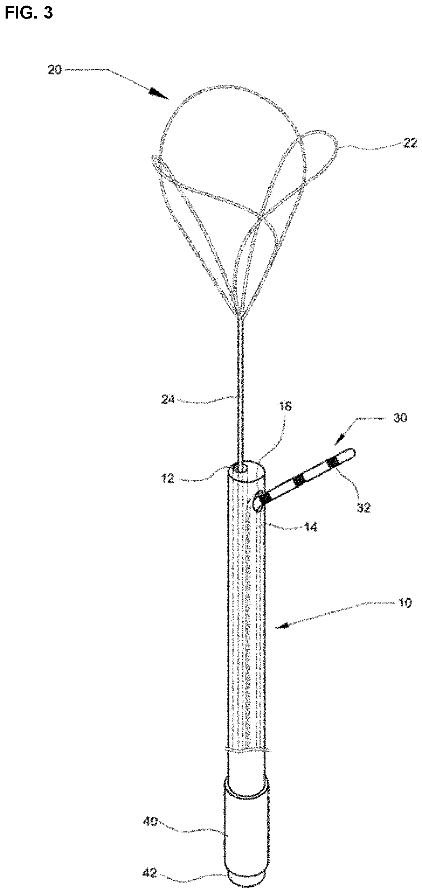

FIG. 3 is a perspective view of a HIS-bundle-detecting snare catheter according to a third exemplary embodiment of the present invention.

In the HIS-bundle-detecting snare catheter according to the third exemplary embodiment of the present invention, the dual lumen catheter 10 includes a hole 18 formed in a side of a distal part, and the hole 18 is coupled to and communicates with a distal end of the second lumen 14.

In other words, the distal end of the second lumen 14 is located on a side of the distal part of the dual lumen catheter 10, and a distal end of the first lumen 12 is located on the distal end of the dual lumen catheter 10.

Accordingly, the snare 20 or the electrical-signal-detecting catheter 30 which is inserted into the second lumen 14 protrudes toward the side of the distal part of the dual lumen catheter 10.

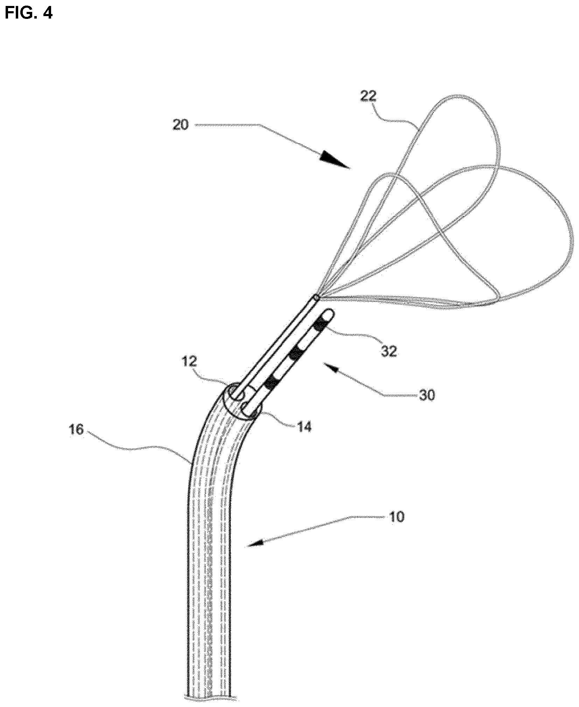

FIG. 4 is a perspective view of a HIS-bundle-detecting snare catheter according to a fourth exemplary embodiment of the present invention.

The HIS-bundle-detecting snare catheter according to the fourth exemplary embodiment of the present invention includes a bending portion 16 on a distal part of the dual lumen catheter 10. The bending portion 16 is formed by bending a distal part of the dual lumen catheter 10 at a certain angle, and the dual lumen catheter 10 includes the bending portion 16 and bends at a certain angle in advance and enters a human body.

The dual lumen catheter 10 according to the fourth exemplary embodiment of the present invention includes the bending portion 16 and includes the position adjustor 40 on a proximal part so as to move a distal part of the dual lumen catheter 10 further toward myocardia. The position adjustor 40 is the same as that described above with reference to FIG. 1 and may move the distal part of the dual lumen catheter 10 leftward or rightward by adjusting the position adjustor 40.

Accordingly, the HIS-bundle-detecting snare catheter according to the present invention may include the dual lumen catheter 10 to move the snare 20 and the electrical-signal-detecting catheter 30 to a position of myocardia desired by a practitioner, may detect an electrical signal of the myocardia to see a position of a His bundle, and may capture and induce the cerclage wire having passed through the His bundle to inferior vena cava.

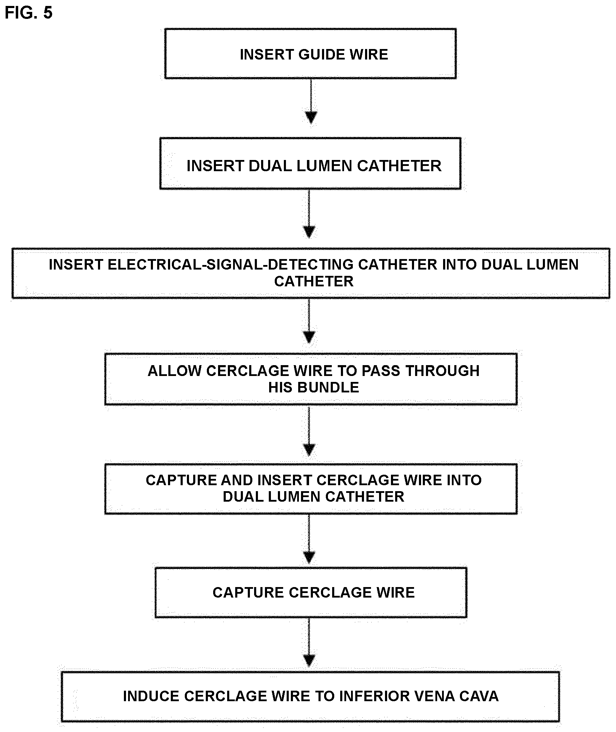

FIG. 5 is a flowchart illustrating a method of performing treatment using the HIS-bundle-detecting snare catheter according to the present invention.

First, a guide wire (not shown) is inserted sequentially into a femoral vein, inferior vena cava, the right atrium, and the right ventricle. Next, the dual lumen catheter 10 enters a human body along the guide wire. Here, as the guide wire is inserted into the dual lumen catheter 10, the guide wire enters the human body along a path through which the guide wire is inserted into the human body. The guide wire is inserted into the first lumen 12 or the second lumen 14 of the dual lumen catheter 10 and it does not matter whether the guide wire is inserted into the first lumen 12 or the second lumen 14. The dual lumen catheter 10 is inserted sequentially into a femoral vein, inferior vena cava, the right atrium, and the right ventricle which is the same path as that of the guide wire so that a distal part thereof is located in the right atrium.

Here, in order to move the distal part of the dual lumen catheter 10 to a position desired by a practitioner, the distal part of the dual lumen catheter 10 may be moved leftward or rightward using the position adjustor 40.

Next, an operation of inserting the electrical-signal-detecting catheter 30 into the dual lumen catheter 10 inserted into the human body is performed. The electrical-signal-detecting catheter 30 may be inserted into the first lumen 12 or the second lumen 14 and may be inserted into the human body to detect an electrical signal of myocardia. Here, the guide wire may be removed from the human body. The electrical-signal-detecting catheter 30 may include a plurality of electrodes to detect electrical signals at a variety of positions of myocardia and may locate a His bundle according to the detected electrical signal. The cerclage wire 50 passes through a position of the His bundle detected using the electrical-signal-detecting catheter 30.

The cerclage wire 50 moves into the human body through superior vein cava and sinus coronaries and passes through the His bundle to guide an end of a lead of a pacemaker to be fixed to the His bundle. Accordingly, when the cerclage wire 50 passes through the His bundle, the end of the lead of the pacemaker which is inserted into the human body along a path of the cerclage wire 50 may be fixed to the His bundle so as to further improve an arrhythmia treatment effect.

The snare 20 is inserted into the dual lumen catheter 10 to capture the cerclage wire 50 having passed through the His bundle. When the snare 20 is pushed to protrude from the distal part of the dual lumen catheter 10, the cerclage wire 50 is located in the capture portion 22 of the snare 20, and then the snare 20 is pulled and inserted into the dual lumen catheter 10, the cerclage wire 50 is inserted, with the snare 20, into the dual lumen catheter 10.

Finally, when the dual lumen catheter 10, the snare 20, and the electrical-signal-detecting catheter 30 are removed from the human body through the inferior vena cava, the cerclage wire 50 may move, with the snare 20, to the inferior vena cava and thus an end thereof may be located outside the human body.

Accordingly, in the HIS-bundle-detecting snare catheter according to the present invention, the electrical-signal-detecting catheter and the snare are inserted into the dual lumen catheter simultaneously to detect an electrical signal of myocardia so as to allow the cerclage wire to pass through a position of myocardia desired by the user and to easily capture and move the cerclage wire having passed through the His bundle to the inferior vena cava.

Although the embodiments of the present invention have been described above with reference to the attached drawings, it can be understood by one of ordinary skill in the art that the present invention may include other detailed forms without departing from the technical concept or essential features thereof. Therefore, it should be understood that the above-described embodiments are exemplary and not limitative in every aspect.

## Claims

1. A HIS-bundle-detecting snare catheter comprising:
a dual lumen catheter comprising a first lumen and a second lumen which are formed therein;
an electrical-signal-detecting catheter inserted into the first lumen or the second lumen to detect an electrical signal of myocardia; and
a snare inserted into the first lumen or the second lumen and comprising one or more annular wire at a distal part,
wherein the electrical-signal-detecting catheter and the snare are inserted into different lumens, respectively.

2. The HIS-bundle-detecting snare catheter of claim 1, wherein the dual lumen catheter comprises a bending portion at the distal part to be bent at a predetermined angle.

3. The HIS-bundle-detecting snare catheter of claim 1, wherein the dual lumen catheter comprises a position adjustor installed on a proximal part so as to adjust a position of the distal part of the dual lumen catheter when the position adjustor is adjusted.

4. The HIS-bundle-detecting snare catheter of claim 1, wherein the dual lumen catheter comprises a hole formed in a side of the distal part, and
wherein the hole is coupled to and communicates with an end of a distal part of the first lumen or the second lumen, and the electrical-signal-detecting catheter or the snare protrudes through the hole.

5. The HIS-bundle-detecting snare catheter of claim 1, wherein the snare comprises:
a capture portion comprising one or more annular wires; and
a body portion having one end coupled to the annular wire and another end passing through the snare lumen and located outside a human body, and
wherein the other end of the body portion is pushed or pulled to allow the capture portion of the snare to be inserted into or protrude from the dual lumen catheter.

6. The HIS-bundle-detecting snare catheter of claim 1, wherein a guide wire is inserted into the first lumen or the second lumen, and
wherein the guide wire is inserted into the human body in advance of the HIS-bundle-detecting snare catheter to allow the HIS-bundle-detecting snare catheter to easily move to the human body.

7. The HIS-bundle-detecting snare catheter of claim 3, comprising a hemostasis valve on an end of a proximal end of the position adjustor to prevent blood flowing into the dual lumen catheter from leaking when the HIS-bundle-detecting snare catheter is inserted into the human body.
